(19) Europäisches Patentamt
European Patent Office
Office européen des brevets

(11) Publication number: **0 067 612**
**A1**

# (12) EUROPEAN PATENT APPLICATION

(21) Application number: 82302824.6

(22) Date of filing: 02.06.82

(51) Int. Cl.³: C 07 D 499/00
A 61 K 31/43
//C07D239/48

(30) Priority: 12.06.81 GB 8118109

(43) Date of publication of application: 22.12.82 Bulletin 82/51

(84) Designated Contracting States: CH DE FR GB IT LI NL

(71) Applicant: BEECHAM GROUP PLC
Beecham House Great West Road
Brentford Middlesex(GB)

(72) Inventor: Driver, Michael John
25 Broadwood Close Roffey
Horsham Sussex(GB)

(72) Inventor: Taylor, Andrew William
7 Doods Road
Reigate Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

## (54) Penicillin derivatives.

(57) A compound of formula (I) or a pharmaceutically acceptable salt or *in vivo* hydrolysable ester thereof:

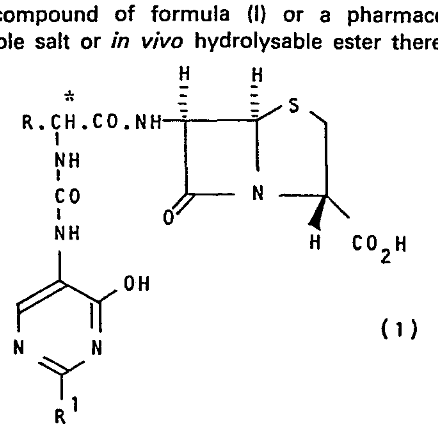

(1)

wherein R is phenyl, 4-hydroxyphenyl, 2- or 3-thienyl, cyclohexyl, cyclohexan-1-yl, cyclohexa-1,4-dien-1-yl, or 3,4-disubstituted phenyl wherein the substituents may be the same or different and are selected from chlorine, hydroxy and methoxy; and $R^1$ represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenylalkylamino group, an acylamino group or an alkyl, aralkyl or arylsulphonyl amino group.

EP 0 067 612 A1

Croydon Printing Company Ltd.

# PENICILLIN DERIVATIVES

This invention relates to a class of penicillin derivatives which have antibacterial activity and are of value in the treatment of infections in animals, including man and poultry, caused by a wide range of organisms, particularly Gram-negative organisms. In particular the invention relates to a class of bis nor penicillin derivatives. The invention also relates to a process for the preparation of such compounds, and to pharmaceutical compositions comprising them.

British Patent Specification No 1,546,622 discloses *inter alia* a class of compounds of the formula (A):

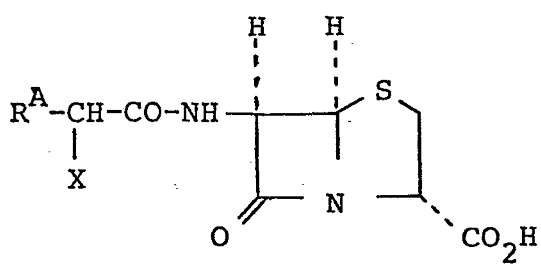

(A)

and pharmaceutically acceptable salts and *in-vivo* hydrolysable esters thereof wherein $R^A$ is a phenyl, 4-hydroxyphenyl, 2-thienyl or 3-thienyl group and X is an amino, hydroxyl, carboxyl or $C_{1-7}$ esterified carboxyl group.

We have now found a class of penicillins lacking the gem-dimethyl groups in the nucleus (referred to as "bis-nor penicillins") which have a high level of antibacterial activity against gram-negative organisms.

According to the present invention there is provided a compound of formula (I) or a pharmaceutically acceptable salt or in vivo hydrolysable ester thereof:

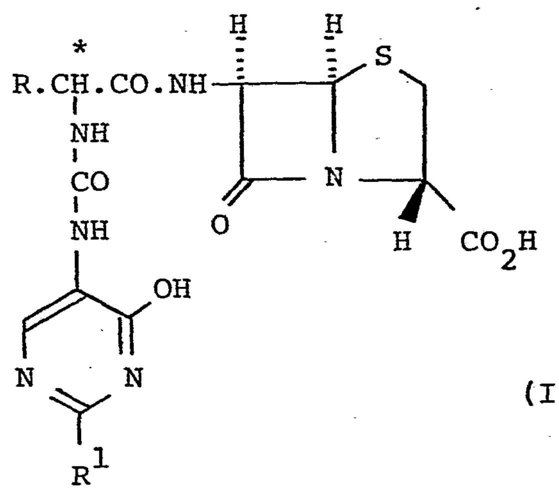

(I)

wherein R is phenyl, 4-hydroxyphenyl, 2- or 3-thienyl, cyclohexyl, cyclohexan-1-yl, cyclohexa-1,4-dien-1-yl, or 3,4-disubstituted phenyl wherein the substituents may be the same or different and are selected from chlorine, hydroxy and methoxy; and $R^1$ represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenylalkylamino group, an

acylamino group or an alkyl, aralkyl or arylsulphonyl amino group.

The compounds of the present invention include the pharmaceutically acceptable esters of compound (I) which hydrolyse readily in the human body to produce the parent acid, for example acyloxyalkyl groups such as acetoxymethyl, pivaloyloxymethyl, α-acetoxyethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl, dimethylaminoethyl, diethylaminomethyl or diethylaminoethyl; and lactone groups such as phthalidyl or dimethoxyphthalidyl.

Suitable salts of the compound of formula (I) include metal salts eg aluminium, alkali metal salts such as sodium or potassium alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-β-phenethylamine, dehydroabietylamine, N,N'-bisdehydroabietylamineethylenediamine, or bases of the pyridine type such as pyridine, collidine or quinoline, or other amines which have been used to form salts with known penicillins.

The carbon atom marked * in formula (I) is asymmetric so that the compounds may exist as two optically active diastereoisomers. In general that

prepared from the D-side chain exhibits the highest antibacterial activity.

In formula (I), the group R is preferably phenyl, 4-hydroxyphenyl, 2-thienyl or 3-thienyl.

Suitably $R^1$ represents a substituted amino group.

More suitably $R^1$ represents an amino group substituted by an optionally substituted phenyl group. The substituent for the phenyl group is preferably in the para position and is selected from amino, alkylamino, $C_{1-6}$alkyl, hydroxy, alkoxy and aminosulphonyl.

Specific compounds within this invention include the following: 6β-[D, 2-{2-anilino-4-hydroxypyrimidyl-5-aminocarbonylamino}-2-phenyl] acetamidobisnor penicillanic acid; 6β-[D,2-{2-(4-aminosulphonyl-anilino)-4-hydroxypyrimidyl-5-aminocarbonylamino}-2(4-hydroxyphenyl)] acetamidobisnor penicillanic acid; and 6β-[D,2-{2-(4-aminosulphonyl-anilino)-4-hydroxypyrimidyl-5-aminocarbonylamino}-2-phenyl] acetamidobisnor penicillanic acid.

The compounds of formula (I) may be prepared by reacting a compound of formula (II):

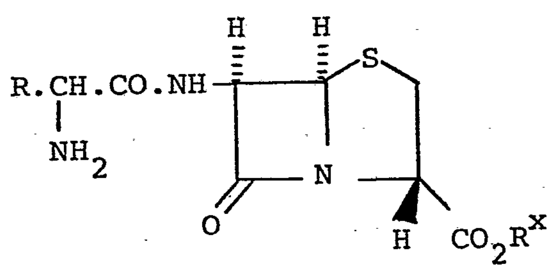

(II)

wherein the amino group is optionally substituted with a group which permits acylation to take place, R is as defined with respect to formula (I) and any reactive

substituents may be protected, and $R^x$ is hydrogen or a carboxyl-blocking group, with an N-acylating derivative of an acid of formula (III).

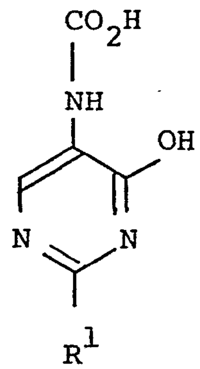

(III)

wherein $R^1$ is as defined with respect to formula (I) above, and any reactive groups may be protected; and thereafter, if necessary, carrying out one or more of the following steps:

(i) removing any carboxyl-blocking group $R^x$

(ii) removing any protecting groups on the side chain group;

(iii) converting the product into a salt or in vivo hydrolysable ester thereof.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-*n*-butyltin, groups of formula $-P.R^aR^b$ wherein $R^a$ is an alkyl, haloalkyl, aryl, aralkyl, alkoxy, haloalkoxy, aryloxy, aralkyloxy or dialkylamino group, $R^b$ is the same as $R^a$ or is halogen

or $R^a$ and $R^b$ together form a ring; suitable such phosphorus groups being $-P(OC_2H_5)_2$, $-P(C_2H_5)_2$,

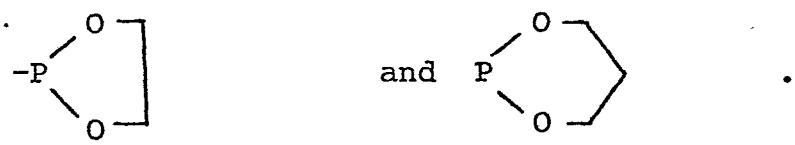

Suitable carboxyl-blocking derivatives for the group $-CO_2R^x$ in formula (II) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with tri-lower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula $-N{=}CHR^o$ where $R^o$ is aryl or heterocyclic, or an _in vivo_ hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

A reactive N-acylating derivative of the acid (III) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be affected in the presence of an acid binding agent for example tertiary amine (such as triethylamine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide - such as ethylene oxide or propylene oxide. The acylation reaction using an acid halide may be carried out at a temperature in the range $-50^{o}C$ to $+50^{o}C$, preferably $-20^{o}C$ to $+20^{o}C$, in aqueous or non-aqueous media such as aqueous acetone, aqueous tetrahydroform, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The intermediate compound of formula (II) may be prepared by reacting a compound of formula (V):

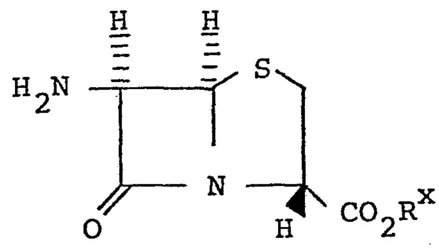

(V)

wherein the amino group is optionally substituted with a group which permits acylation to take place and $R^x$ is as defined with respect to formula (II) above, with an N-acylating derivative of an acid of formula (VI):

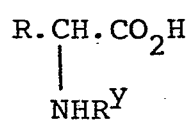

(VI)

wherein R is as defined with respect to formula (I) and any reactive groups therein may be protected and $R^Y$ is an amino-protecting group; and thereafter removing protection group $R^Y$.

Suitable N-acylating derivatives, carboxyl protecting groups and reaction conditions include those described hereinbefore.

Suitable amino-protecting groups $R^Y$ are those well-known in the art which may be removed under conventional conditions without disruption of the remainder of the molecule.

The starting material of formula (V) is disclosed in British Patent No. 1,546,622.

The compounds of formula (I) may also be prepared by reacting a compound of formula (V) as described hereinbefore with an N-acylating derivative of an acid of formula (VII):

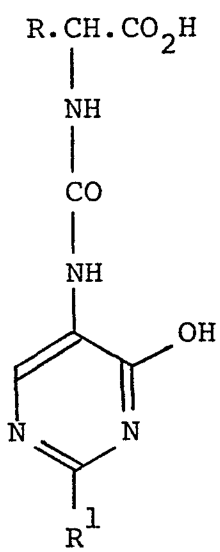

(VII)

wherein R and $R^1$ are as defined with respect to formula (I) and any reactive groups therein may be protected; and thereafter, if necessary, carrying out one or more of the following steps:

(i) removing any carboxyl-blocking group $R^x$;
(ii) removing any protecting groups on the side chain group;
(iii) converting the product into a salt or in vivo hydrolysable ester thereof.

The antibiotic compounds according to the invention may be formulated for administration in any convenient way for use in human or veterinary medicine, by analogy with other antibiotics, and the invention therefore includes within its scope pharmaceutical compositions comprising

a compound of formula (I) above together with a pharmaceutical carrier or excipient.

The compositions may be formulated for administration by any route, such as oral topical or parenteral. The compositions may be in the form of tablets, capsules, powders, granules, lozenges, creams or liquid preparations, such as oral or sterile parenteral solutions or suspensions.

Tablets and capsules for oral administration may be in unit dose presentation form, and may contain conventional excipients such as binding agents, for example, syrup, acacia, gelatin, sorbitol, tragacanth, or polyvinylpyrollidone; fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxylmethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate

or sorbic acid, and if desired conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3,000 mg per day, for instance 1,500 mg per day depending on the route and frequency of administration.

The compound of formula (I) may be the sole therapeutic agent in the compositions of the invention or a combination with other antibiotics and/or a β-lactamase inhibitor may be employed.

Advantageously, the compositions also comprise a compound of formula (VIII), or a pharmaceutically acceptable salt or ester thereof:

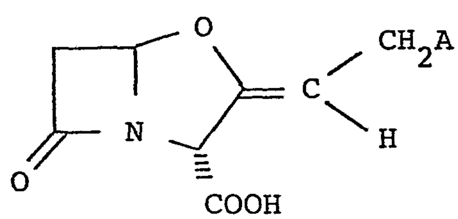

(VIII)

wherein A is hydroxyl, substituted hydroxyl, thiol, substituted thiol, amino, mono- or di-hydrocarbyl-substituted amino, or mono- or di-acylamino.

The following Examples illustrate the preparation of some of the compounds of this invention.

Example 1

D, 2-(2-Anilino-4-hydroxypyrimidyl-5-aminocarbonylamino)-2-phenyl acetic acid (1)

2-Anilino-4-hydroxy-5-aminopyrimidine (0.2 g, 1 mmol) dissolved in dry THF (25 ml) with $Et_3N$ (0.14 ml, 1 mmol) was cooled at $0^o$ under $N_2$ and treated, dropwise, with a solution of phosgene in toluene (1 ml of 12.5% solution). The mixture was allowed to warm to room temperature, stirred for one hour and then concentrated to low bulk twice diluting each time with dry THF (15 ml).

D-Phenylglycine (0.15 g, 1 mmol) in 80% THF at pH 9 was cooled to $0^o$ and treated with the above suspension. The pH was maintained at 8 with $Et_3N$, the solution allowed to warm to room temperature and stirred for 2 hours. THF was then evaporated, the aqueous phase washed with ethyl acetate, acidified to pH 1.5 and the precipitate filtered and dried (0.24 g, 63%) $\nu_{max}$ (Nujol) 1720, 1640 and 1540 $cm^{-1}$, $\delta$ (DMSO-$d^6$) 8.4 (3H, s, pyrimidine + 2 exchangeable protons), 8.0-6.9 (13H, m, aryl protons + 3 exchangeable protons) and 5.4 (1H, d, J 7Hz, CH) ppm.

Benzyl 6β-[D,2-{2-anilino-4-hydroxypyrimidyl-5-aminocarbonylamino}-2-phenyl]acetamidobisnorpenicillanate (2)

The glycine derivative (1) (0.2 g, 0.53 mmol) in dry DMF (4 ml) was cooled to $0^o$ under $N_2$ and treated firstly with a solution of dicyclohexylcarbodiimide (0.12 g, 0.58 mmol) in DCM (3 ml) and then a solution of benzyl 6β-aminobisnorpenicillinate (3) (0.12 g,

0.42 mmol) in dry DCM (5 ml). The solution was stirred for 45 minutes at $0^o$ and then 90 minutes at room temperature. Acetic acid (3 drops) was added, the mixture evaporated to near dryness and the residue suspended between ethyl acetate and dilute sodium bicarbonate solution. The aqueous phase was discarded while the organic layer was washed with dilute hydrochloric acid, brine then dried and evaporated to give a colourless solid (0.24 g, 87%).

$\nu_{max}$ (Nujol 3325, 1780, 1735 and 1630 $cm^{-1}$. $\delta$($d^6$-DMSO+$D_2O$) 8.14 (1H, s, pyrimidine proton), 7.58-7.24 (15H, m, aryl protons), 5.52 (1H, d, J 4Hz, H-6), 5.50 (1H, s, CH), 5.18 (4H, m, H-3, H-5, $CH_2$) and 3.42 (2H, m, H-2 x 2) ppm.

Sodium 6β-[D,2-{anilino-4-hydroxypyrimidyl-5-aminocarbonylamino}-2-phenyl]acetamidobisnorpenicillanate

The ester (2) (0.1 g, 0.16 mmol) in THF/MeOH (10 ml:15 ml) was filtered, treated with 10% Pd/C (0.1 g) and hydrogenated for one hour. Tlc showed starting material remaining so more catalyst (0.05 g) was added and hydrogenolysis continued for a further 2 hours. The mixture was filtered through celite and evaporated to give a white residue (0.05 g). The crude product was treated with dilute sodium bicarbonate solution (4 ml), insoluble material was filtered off, the filtrate was acidified to pH 1.5 and extracted into ethyl acetate (3 x 10 ml). The combined organic extracts were dried ($MgSO_4$) and evaporated to give a white solid (0.035 g). This free acid was converted into the corresponding sodium salt using sodium ethylhexanoate (1.1 eq) in methylisobutyl ketone.

$\nu$ max (Nujol) 3300 (br), 1780, 1690, 1670, and 1600 $cm^{-1}$.

δ ($CD_3OD$/DMSO - $d^6$) 8.15 (1H,s,pyrimidine proton), 7.72- 7.12 (10H,m, aryl protons), 5.52 (2H,m,H-6,α-H), 5.18 (1H,d,*J* 4Hz,H-5), 4.80 (1H,H-3 masked by solvent) and 3.51-3.30 (2H,m,H-2 x 2) ppm.

Example 2

D,2-[2-(4-Aminosulphonylanilino)-4-hydroxypyrimidyl-5-aminocarbonylamino)-2-(4-hydroxyphenyl)acetic acid (3)

2-(4-Aminosulphonylanilino)-4-hydroxy-5-aminopyrimidine (0.56 g, 2 mmol) in dry THF (250 ml) was cooled to $0^{o}$ and treated with, firstly, triethylamine (0.28 ml, 2 mmol) and then phosgene (2 ml of 12.5% solution). The mixture was stirred for one hour and then concentrated to ca 40 ml. This suspension was added to a stirred solution of D,p-hydroxyphenylglycine (0.32 g, 2 mmol) in 50% THF at pH 9.5. Stirring was continued for two hours at $21^{o}$ while the pH was maintained at 9.0 with dilute sodium hydroxide solution. THF was evaporated and the aqueous residue washed with ethyl acetate and then filtered. The filtrate was acidified to pH 1.5 and the precipitate collected by filtration. This material was purified using column chromatography on silica eluting with mixtures of ethyl acetate, isopropyl alcohol and water to give a yield of ca 20%.
$\nu_{max}$ (Nujol) 1720, 1680-1640 (br), 1540 and 1160cm$^{-1}$
$\delta$ ($d^6$DMSO+$D_2O$) 8.4 (1H, s, pyrimidine proton), 7.9 (4H, s, p-aminosulphonylphenyl protons), 7.4 and 6.9 (4H, ABq, p-hydroxyphenyl protons) and 5.3 (1H, s, CH) ppm.

Benzyl 6β-[D,2-{2-(4-aminosulphonylanilino)-4-hydroxypyrimidyl-5-aminocarbonylamino}-2-(4-hydroxyphenyl)] acetamidobisnorpenicillanate (4)

The title compound was prepared in an analogous manner to that discribed for (2) above. Crude yield (1 mmol scale) (0.35 g, 35%). Purification was effected by column chromatography on silica eluting with ethyl acetate, iso-propanol, methanol and water mixtures.

$\nu_{max}$ (Nujol) 3320, 1775, 1735, 1680 (sh), 1660 and 1160 $cm^{-1}$, $\delta$ ($CD_3OD/d^6DMSO$) 8.3 (1H, s, pyrimidine proton), 7.8 (4H, s, p-aminosulphonylphenyl protons), 7.5 (7H, m, benzyl + low field p-hydroxyphenyl protons), 6.9 (2H, high field p-hydroxyphenyl protons), 5.8-5.1 (6H, m, H-6, H-5, H-3, $\alpha$-H and $CH_2$) and 3.5 (2H, m, H-2 x 2) ppm.

Sodium 6β-[D,2-{2-(4-aminosulphonylanilino)-4-hydroxypyrimidyl-5-aminocarbonylamino}-2(4-hydroxyphenyl(]acetamidobisnor penicillanate

Deprotection of (4) and conversion to the corresponding sodium salt was effected in an analogous manner as described above for (2). The product has ν max (Nujol) 3400-3250 br, 1770,1700, 1670, 1590 and 1170 $cm^{-1}$, δ($CD_3OD$/DMSO - $d^6$) 8.30 (1H,s,pyrimidine proton), 7.76 (4H,s,p-sulphonamidophenyl protons), 7.22 and 6.85 (4H,ABq,p-hydroxyphenyl protons), 5.54-5.50 (2H,m,H-6 and αH), 5.20-5.09 (1H,m,H-5), 4.85(1H,H-3, masked by solvent) and 3.44-3.30 (2H,m,H-2x2) ppm.

Example 3

Benzyl 6β-[D,2-(4-nitrobenzyloxycarbonylamino)-2-phenyl]-acetamidobisnorpenicillanate (5)

D,2-(4-Nitrobenzyloxycarbonylamino)-2-phenylacetyl chloride (2 mmol) was prepared from the corresponding acid by treatment with oxalyl chloride in dry DCM with DMF (2 drops) and has $\nu_{max}$ 3430, 1790, 1725, 1520, 1495 and 1350 $cm^{-1}$. This acid chloride, as a solution in dry DCM (5 ml), was added dropwise to a solution of benzyl 6β-aminobisnorpenicillanate (0.54 g, 2 mmol) in DCM (10 ml) at $0^{o}$ in the presence of pyridine (0.2 ml, 1.3 eq). The mixture was allowed to warm to room temperature and stirred for 2 hours. Work-up gave a gum which was dissolved in a little ethyl acetate, triturated with ethanol and stored at $0^{o}$ overnight. The precipitated solid was collected by filtration, washed with ethanol and dried (0.72 g, 68%), mp 127-128$^{o}$. $\nu_{max}$ (DCM) 3400, 1795, 1740, 1690, 1525, 1500 and 1450 $cm^{-1}$, δ ($CDCl_3$, 250 MHz) 8.18 (2H, d, J 8Hz, low field p-$NO_2$ benzyl protons), 7.48 (2H, d, J 8Hz, remaining p-$NO_2$ benzyl protons), 7.40-7.29 (10H, m, aryl protons), 6.43 (1H, d, J 8Hz, CONH), 6.18 (1H, d, J 5Hz, CHNH), 5.63 (1H, dd, J 4Hz and 8Hz, H-6), 5.20-5.08 (3H, m, $CH_2$ and H-5), 4.94 (1H, dd, J 7Hz and 3Hz, H-3) and 3.32 (2H, ddd, J 11Hz, 7Hz and 3Hz, H-2 x 2) ppm.

Bisnorampicillin (6)

The diprotected bisnorpenicillin (5) (0.44 g, 0.75 mmol) in THF/EtOH (7 ml:4 ml) was treated with 10% Pd/C (0.4 g) and hydrogenated for two hours. Additional catalyst (0.4 g) was added and hydrogenation continued for three hours when the mixture was filtered through celite, washing well with aqueous methanol. The filtrate was evaporated, triturated with ether, filtered and dried to give a pale yellow solid (0.2 g, 83%) $\nu_{max}$ (Nujol) 3300 (br) 1770, 1680 (sh) 1660 and 1600 $cm^{-1}$ $\delta$ ($CD_3OD$) 7.4 (5H, m, aryl protons), 5.6-5.2 (3H, m, H-6, H-5 and α-H), 4.8 (1H, m, H-3) and 3.5 (H-2 absorptions masked by solvent) ppm.

6β-[D,2-{2-(4-Aminosulphonylanilino)-4-hydroxypyrimidyl-5-aminocarbonylamino}-2-phenyl]acetamidobisnor penicillanic acid, sodium salt

2-(4-Aminosulphonylanilino)-4-hydroxy-5-amino-pyrimidine (0.17 g, 0.6 mmol) in THF (100 ml) was cooled to $0^{o}$ and treated with triethylamine (0.084 ml) and then phosgene (0.6 ml of 12.5% solution). The mixture was allowed to warm to $21^{o}$, stirred for 1h and then twice evaporated to ca 2 ml, diluting each time with fresh dry THF (15 ml). This mixture was then added, at $0^{o}$, to a solution of bisnorampicillin (0.17 g, 0.53 mmol) in 80% tetrahydrofuran at pH 8.

The solution was allowed to warm to 21° and stirring was continued for 2h while the pH was maintained at 7.5-8.0 with triethylamine. Tetrahydrofuran was then evaporated, the residue washed with ethyl acetate, acidified to pH 1.5 and extracted with 10% n-butanol in ethyl acetate (4 x 25 ml). The combined organic extracts were dried and evaporated to give a white solid which was treated with sodium ethylhexanoate in methylisobutyl ketone to give the corresponding sodium salt (0.16 g, 34%) which may be purified using column chromatography on silica eluting with ethyl acetate, <u>iso</u>-propanol water mixtures.

$\nu_{max}$ (nujol) 3300 (br), 1770, 1700, 1670, 1580 and 1160 $cm^{-1}$;

$\delta$ ($CD_3OD$/DMSO - $d^6$) 8.23 (1H,s,pyrimidine proton), 7.78 (4H,s,p-sulphonamidophenyl protons), 7.54-7.21 (5H,m,phenyl protons), 5.53-5.49 (2H,m,H-6,α-H), 5.24-5.07 (1H,m,H-5), 4.76 (1H,H-3,masked by solvent) and 3.42-3.30 (2H,m,H-2 x 2) ppm.

Example 4

Benzyl 6β-[D,2-(4-nitrobenzyloxycarbonylamino)-2-(4-benzyloxycarbonyloxyphenyl)]acetamidobisnorpenicillanate (7)

The title compound was prepared in an analogous manner to that described for (5) above by treating benzyl 6β-aminobisnorpenicillanate with D,2-(4-nitrobenzyloxycarbonylamino)-2-(4-benzyloxycarbonyloxy)phenyl acetyl chloride (1 eq) in the presence of pyridine (1.3 eq). Work-up, chromatography and crystallisation gave the product as a colourless solid in 49% yield m.p. 92°. $\nu_{max}$ (Nujol) 3350, 1785, 1760, 1740, 1680, 1520, 1350, 1260 and 1220 $cm^{-1}$. δ ($CDCl_3$) 8.18 (2H, d, J 8Hz, low field p-$NO_2$ phenyl protons), 7.50-7.28 (14H, m, aryl protons), 7.18 (2H, d, J 8Hz, high field p-benzyloxycarbonyloxy-phenyl protons), 6.74 (1H, d, J 8Hz, CONH), 6.23 (1H, d, J 6Hz, CH-NH), 5.58 (1H, dd, J 8Hz and 4Hz, H-6), 5.31-5.05 (8H, m, $CH_2$x 3, H-5 and α-H), 4.93 (1H, dd, J 6Hz and 3Hz, H-3) and 3.40 and 3.23 (2H, m, H-2 x 2) ppm.

Bisnoramoxycillin (8)

The triprotected bisnorpenicillin (7) (0.2 g, 0.27 mmol) in THF:EtOH:$H_2$O.DCM (5 ml:5 ml:2 ml:10 ml) was hydrogenated with 10% Pd/C (0.2 g) for two hours. Additional catalyst (0.2 g) was then added and hydrogenation continued for a further two hours. Filtration through celite followed by evaporation of the filtrate gave a yellow foam (0.09 g, 100%) $\nu_{max}$ (Nujol) 1770, 1680, 1610 $cm^{-1}$ δ (CDOD) 7.2, 6.8 (4H, ABq, p-hydroxyphenyl protons), 5.6-5.1 (4H, m, H-6, H-5, H-3 and α-H), 3.4 (2H, m, H-2 x 2) ppm.

6β-[D,2-{2-(4-Aminosulphonylanilino)-4-hydroxypyrimidyl-5-aminocarbonylamino}-2-(4-hydroxyphenyl)]acetamido-bisnorpenicillanic acid, sodium salt

The title compound was prepared in an analogous manner to that described in example 3. The product had $\nu_{max}$ (Nujol) 1770, 1680-1650 (br), 1600 and 1160 $cm^{-1}$ δ (DMSO-$d^6$/$CD_3OD$) 8.2 (1H, s, pyrimidine proton), 7.8 (4H, s, p-aminosulphonylphenyl protons), 7.2 and 6.8 (4H, ABq, p-hydroxyphenyl protons), 5.6 (1H, d, J 4Hz, H-6), 5.45-5.05 (3H, m, H-5, H-3, α-H), 3.4 (H-2 absorptions masked by solvent) ppm.

BIOLOGICAL DATA

The antibacterial activities of compounds of the present invention (MIC µg/ml)

| Organism | Compound of Example Number 3 Agar | Compound of Example Number 4 Agar |
|---|---|---|
| E. coli ESS | 0.05 | 0.05 |
| E. coli JT 4 | >100.0 | 100.0 |
| E. coli JT 425 | 5.0 | 2.5 |
| E. coli NCTC 10418 | 0.5 | 0.25 |
| Ps. aeruginosa NCTC 10662 | 5.0 | 2.5 |
| Ps. aeruginosa NCTC 10662 $10^{-2}$ | 2.5 | 0.5 |
| Ps. aeruginosa Dalgleish $10^{-2}$ | 50.0 | 25.0 |
| S. marcescens US 32 | 2.5 | 1.0 |
| K. aerogenes A | 0.2 | 0.25 |
| E. cloacae N1 | 1.0 | 2.5 |
| P. mirabilis C 977 | 0.2 | 2.5 |
| P. mirabilis 889 | >100.0 | >100.0 |
| P. rettgeri | 1.0 | 2.5 |
| S. aureus Oxford | 1.0 | 2.5 |
| S. aureus Russell | 50.0 | 25 |
| N. catarrhalis 1502 | 0.05 | 0.05 |
| S. faecalis I | 25 | 25 |
| S. pyogenes CN 10 | 0.2 | 0.1 |

Claims

1. A compound of formula (I) or a pharmaceutically acceptable salt or in vivo hydrolysable ester thereof:

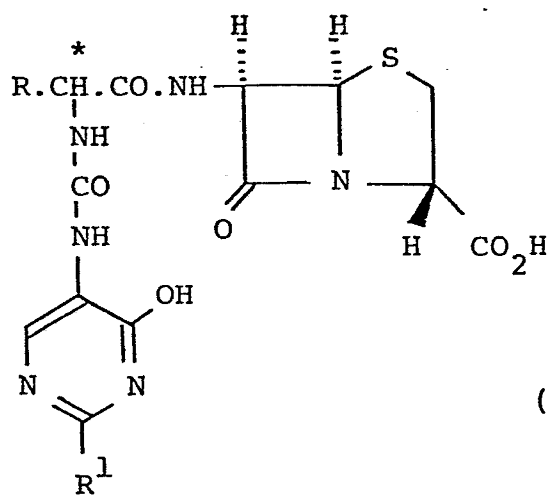

(I)

wherein R is phenyl, 4-hydroxyphenyl, 2- or 3-thienyl, cyclohexyl, cyclohexan-1-yl, cyclohexa-1,4-dien-1-yl, or 3,4-disubstituted phenyl wherein the substituents may be the same or different and are selected from chlorine, hydroxy and methoxy; and $R^1$ represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenylalkylamino group, an acylamino group or an alkyl, aralkyl or arylsulphonyl amino group.

2. A compound as claimed in claim 1 wherein the carbon atom marked in formula (I) is in the D configuration.

3. A compound as claimed in claim 1 or claim 2 wherein R is phenyl, 4-hydroxyphenyl, 2-thienyl or 3-thienyl.

4. A compound as claimed in any one of claims 1 to 3 wherein $R^1$ represents a substituted amino group.

5. A compound as claimed in any one of claims 1 to 4 wherein $R^1$ represents an amino group substituted by an optionally substituted phenyl group.

6. A compound as claimed in claim 5 wherein the substituent for the phenyl group within $R^1$ is in the para position and is selected from amino, alkylamino, $C_{1-6}$ alkyl, hydroxy, alkoxy and aminosulphonyl.

7. A compound as claimed in claim 1 selected from the following or a pharmaceutically acceptable salt or in vivo hydrolysable ester thereof; 6β-[D,2-{2-anilino-4-hydroxypyrimidyl-5-aminocarbonylamino}-2-phenyl]acetamidobisnor penicillanic acid; and 6β-[D,2-{2-(4-aminosulphonylanilino)-4-hydroxypyrimidyl-5-aminocarbonylamino}-2(4-hydroxyphenyl] acetamidobisnor penicillanic acid; and 6β-[D,2-{2-(4-aminosulphonylanilino)-4-hydroxypyrimidyl-5-aminocarbonylamino}-2-phenyl]acetamidobisnor penicillanic acid.

8. A process for the preparation of a compound as claimed in claim 1 which process comprises:

a) reacting a compound of formula (II):

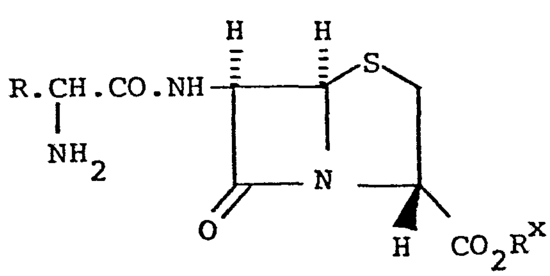

(II)

wherein the amino group is optionally substituted with a group which permits acylation to take place, R is as defined with respect to formula (I) and any reactive substituents may be protected, and $R^X$ is hydrogen or a carboxyl-blocking group, with an N-acylating derivative of an acid of formula (III).

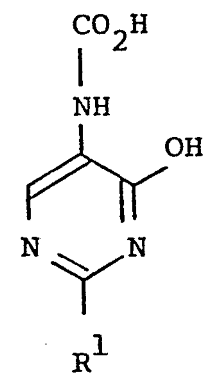

(III)

wherein $R^1$ is as defined with respect to formula (I) above, and any reactive groups may be protected or

b) reacting a compound of formula (V):

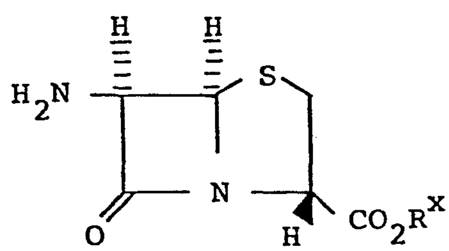

(V)

wherein the amino group is optionally substituted with a group which permits acylation to take place and $R^X$ is as defined with respect to formula (II) above, with an N-acylating derivative of an acid of formula (VII):

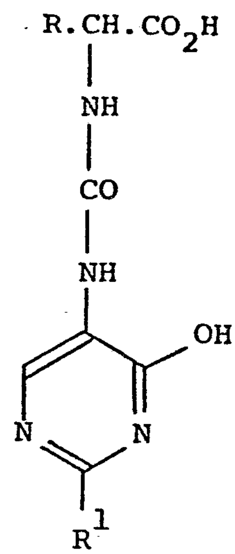

(VII)

wherein R and $R^1$ are as defined with respect to formula (I) and any reactive groups therein may be protected; and after any of processes a) and b), if necessary, carrying out one or more of the following steps:

(i) removing any carboxyl-blocking group $R^x$;
(ii) removing any protecting groups on the side chain group;
(iii) converting the product into a salt or in vivo hydrolysable ester thereof.

9. A pharmaceutical composition comprising a compound as claimed in any one of claims 1 to 7 together with a pharmaceutical carrier or excipient.

10. A pharmaceutical composition as claimed in claim 9 which further comprises a β-lactamase inhibitor.

0067612

European Patent Office

# EUROPEAN SEARCH REPORT

Application number

EP 82 30 2824

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 487 074 (J.C. SHEEHAN)<br>* abstract and claims 1-12 *<br>--- | 1,9 | C 07 D 499/00<br>A 61 K 31/43 //<br>C 07 D 239/48 |
| A | EP-A-0 003 814 (DR. KARL THOMAE)<br>* claims *<br>--- | 1-6,8,9 | |
| P,A | EP-A-0 043 205 (BEECHAM)<br>* claims *<br>----- | 1-6,8-10 | |
| | | | TECHNICAL FIELDS SEARCHED (Int. Cl. 3) |
| | | | C 07 D 499/00<br>A 61 K 31/00 |

The present search report has been drawn up for all claims

| Place of search | Date of completion of the search | Examiner |
|---|---|---|
| THE HAGUE | 25-08-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82